# EUROPÄISCHE PATENTSCHRIFT

(11) **EP 1 720 662 B1**
(45) Veröffentlichungstag und Bekanntmachung des Hinweises auf die Patenterteilung: **13.04.2016**
(21) Anmeldenummer: 05715510.3
(22) Anmeldetag: 24.02.2005
(51) Int. Cl.: B05B 11/00, A61M 15/00, A61M 16/00

(54) **ZERSTUBER MIT EINER BERWACHUNGSEINRICHTUNG ZUR ZÄHLUNG VON BETÄTIGUNGEN DES ZERSTÄUBERS**
ATOMIZER PROVIDED WITH A MONITORING DEVICE FOR COUNTING ACTUATIONS OF THE ATOMIZER
PULVERISATEUR POURVU D'UN DISPOSITIF DE SURVEILLANCE SERVANT A COMPTER LES ACTIONNEMENTS DU PULVERISATEUR

(30) Priorität: 24.02.2004 DE 102004009435
(43) Veröffentlichungstag der Anmeldung: 15.11.2006
(73) Patentinhaber: Boehringer Ingelheim International GmbH, 55216 Ingelheim (DE)
(72) Erfinder: WUTTKE, Gilbert, 44149 Dortmund (DE); KAULMANN, Stefan, 71642 Ludwigsburg (DE); KUNZE, Hubert, 44227 Dortmund (DE); FIOL, Andreas, 22850 Norderstedt (DE); ZIERENBERG, Bernd, 55411 Bingen (DE); NOEHL, Klaus, 55218 Ingelheim (DE); GESER, Johannes, 55218 Ingelheim (DE)
(74) Vertreter: Von Rohr Patentanwälte Partnerschaft mbB
(86) Internationale Anmeldenummer: PCT/EP2005/001942
(87) Internationale Veröffentlichungsnummer: WO 2005/080001

(56) Entgegenhaltungen:
- EP-A- 0 775 499
- WO-A-02/053295
- US-A- 5 209 375
- US-A- 5 392 768
- US-A- 5 482 030
- US-A1- 2002 066 752
- US-A1- 2003 160 061
- US-B1- 6 234 366
- US-B1- 6 510 847

## Beschreibung

Der vorliegende Erfindung betrifft einen Zerstäuber gemäß dem Oberbegriff des Anspruchs 1.

Ausgangspunkt der vorliegenden Erfindung ist ein unter dem Handelsnamen "Respimat" von der Boehringer Ingelheim KG angebotener Zerstäuber in Form eines Inhalators, wie im Grundprinzip in der WO 91/14468 A1 und in konkreter Ausgestaltung in der WO 97/12687 A1 (Fig. 6a, 6b) sowie in Fig. 1 und 2 der anliegenden Zeichnung dargestellt. Der Zerstäuber weist als Reservoir für ein zu zerstäubendes Fluid einen einsetzbaren Behälter mit dem Fluid und einen Druckerzeuger mit einer Antriebsfeder zur Förderung und Zerstäubung des Fluids auf. Durch Drehen eines Betätigungsteils in Form eines Gehäuseunterteils des Zerstäubers ist die Antriebsfeder spannbar und Fluid in eine Druckkammer des Druckerzeugers saugbar. Nach manueller Betätigung eines Sperrelements wird das Fluid in der Druckkammer von der Antriebsfeder unter Druck gesetzt und zerstäubt, also unter Bildung eines Aerosols ausgegeben. Beim Spannen einerseits und der dann folgenden Zerstäubung andererseits führt der Behälter jeweils eine Hubbewegung aus. Der Zerstäuber weist eine mechanische Überwachungseinrichtung auf, die zur Zählung von Betätigungen des Zerstäubers das Drehen des Betätigungsteils erfaßt. Der bekannte Zerstäuber arbeitet ausschließlich mechanisch, d. h. ohne Treibgas und ohne Elektrik.

Zur Vervollständigung der Offenbarung der vorliegenden Patentanmeldung wird vorsorglich auf den kompletten Offenbarungsgehalt sowohl der WO 91/14468 A1 als auch der WO 97/12687 A1 verwiesen. Generell bezieht sich die dortige Offenbarung bevorzugt auf einen Zerstäuber mit einem Federdruck von 5 bis 60 MPa, bevorzugt 10 bis 50 MPa auf das Fluid, mit Volumina pro Hub von 10 bis 50 µl, bevorzugt 10 bis 20 µl, ganz bevorzugt etwa 15 µl pro Hub, Teilchengrößen von bis zu 20 µm, bevorzugt 3 bis 10 µm. Ferner bezieht sich die dortige Offenbarung bevorzugt auf einen Zerstäuber mit zylinderähnlicher Form und einer Größe von etwa 9 cm bis etwa 15 cm in der Länge und etwa 2 cm bis etwa 5 cm in der Breite sowie von einer Düsen-Strahlfächerung von 20° bis 160°, bevorzugt von 80° bis 100°. Derartige Werte gelten auch für den Zerstäuber nach der Lehre der Erfindung als besonders bevorzugte Werte.

Bekannt ist ferner eine Vorrichtung zum Erfassen der Betätigung eines Spenders, wobei ein Austragsförderer durch eine Hubbewegung zwischen einem Betätigungselement und einem Medienbehälter betätigt wird und im Betätigungselement ein Schalter zur Erfassung einer Betätigung und Erzeugung eines elektrischen Zählsignals angeordnet ist (DE 100 65 160 A1). Der Schalter wird hier bei der linearen Hubbewegung nicht unmittelbar vom Behälter, sondern von einer Befestigungsschraube der Vorrichtung betätigt, so daß bei einer Betätigung auch bei nicht eingesetztem Behälter ein Zählsignal erzeugt wird.

Die DE 100 61 723 C2 offenbart ein mechanisches Zählwerk zum Zählen dosierter Abgaben flüssiger, pastöser oder fester Produkte, insbesondere Medikamente, aus einem Vorratsbehälter, insbesondere einem Aerosolbehälter. Vorzugsweise wird eine lineare Bewegung des Aerosolbehälters gezählt.

Bekannt ist des weiteren eine Austragsteuerung für einen Medien-Spender (DE 198 07 921 A1). Diese Steuerung umfaßt einen Speicher und eine Intervall-Schaltung, welche einen möglichen Betätigungshub nur zu bestimmten Zeiten freigibt und sonst sperrt. Der Speicher ist mit einem Computer so programmierbar, daß die Sperre nur zu bestimmten Zeiten freigegeben wird, wofür ein Programm ein Zeitschaltglied umfaßt. Eine Anzeige zeigt dem Benutzer an, wann eine Mediendosis appliziert werden soll und wann nicht. Der Speicher kann diese Anwendungen erfassen, wonach sie jederzeit mit einem Computer auf einem Bildschirm erkennbar gemacht werden können. Zur Programmierung und Abfrage des Speichers bzw. zur Aufladung eines Energiespeichers kann die Steuerung einen von außen zugänglichen elektrischen Anschluß für einen entsprechenden Stecker aufweisen.

Der vorliegenden Erfindung liegt die Aufgabe zugrunde, einen Zerstäuber der eingangs genannten Art mit einer verbesserten Überwachungseinrichtung anzugeben, insbesondere wobei eine gesteigerte Benutzungssicherheit und ggf. eine weitergehende Information des Benutzers und/oder eine Benutzerüberwachung ermöglicht wird bzw. werden.

Die obige Aufgabe wird durch einen Zerstäuber gemäß Anspruch 1 gelöst. Vorteilhafte Weiterbildungen sind Gegenstand der Unteransprüche.

Gemäß einem ersten Aspekt der vorliegenden Erfindung ist die Überwachungseinrichtung an einem lösbaren Gehäuseteil des Zerstäubers angeordnet, insbesondere fest mit diesem verbunden, vorzugsweise in dieses eingegossen. Dies gestattet ein einfaches Lösen der Überwachungseinrichtung zusammen mit dem Gehäuseteil vom Zerstäuber, so daß die Überwachungseinrichtung sehr einfach - getrennt bzw. unabhängig vom Zerstäuber - eingeschaltet, programmiert, initialisiert und/oder ausgelesen werden kann und/oder so daß sehr einfach ein vollständiger Austausch der Überwachungseinrichtung zusammen mit dem Gehäuseteil oder eine Nachrüstung eines Zerstäubers mit einer Überwachungseinrichtung bei entsprechender Kompatibilität des Gehäuseteils ermöglicht wird.

Ein weiterer, auch unabhängig realisierbarer Aspekt der vorliegenden Erfindung liegt darin, daß ein tatsächlicher Austrag von Fluid erfaßt und insbesondere elektronisch als Betätigung des Zerstäubers gezählt wird. Dies gestattet eine verbesserte Überwachung sowie eine verbesserte Benutzungssicherheit und Benutzerführung.

Ein tatsächlicher Austrag wird dadurch detektiert bzw. erfaßt, indem unmittelbar eine Bewegung, vorzugsweise ein Hub, des Behälters erfaßt wird, daß ein Aufnahmesensor einen durch das Inhalieren hervorgerufenen Zuluftstrom detektiert und/oder daß ein Spraysensor eine Erzeugung von zerstäubtem Fluid bzw. Aerosol, insbesondere im Bereich eines Mundstücks, detektiert. Dementsprechend ist mit wesentlich größerer Sicherheit feststellbar, ob tatsächlich ein Austrag von Fluid bzw. tatsächlich ein Inhalieren erfolgt ist. Vorzugsweise erfolgt die Überwachung nicht nur qualitativ, sondern auch quantitativ.

Gemäß einer Variante ist vorgesehen, daß die Überwachungseinrichtung mittels des Aufhahmesensors ein ausreichend starkes und/oder andauerndes Inhalieren des von dem Zerstäuber zerstäubten Fluids erfaßt und insbesondere speichert und/oder als (erfolgreiche) Betätigung des Zerstäubers bzw. Einnahme des Fluids zählt. Dies ist einer sichereren Bedienung und besseren Überwachung zuträglich.

Besonders bevorzugt ist die Überwachungseinrichtung des Zerstäubers mit einem Zeitgeber und einem Speicher versehen, so daß Anzahl und Zeitpunkt von Betätigungen des Zerstäubers erfaßbar und speicherbar sind und/oder so daß eine wiederholte Betätigung innerhalb einer vorbestimmte Mindestzeitdauer blockierbar und/oder so daß vorzugsweise nach Ablauf einer vorbestimmten Höchstzeitdauer ein Erinnerungssignal vorzugsweise zur erneuten Betätigung ausgebbar oder anzeigbar ist.

Bei Erfassung und Speicherung der Anzahl und Zeitpunkte des tatsächlichen Austrags von Fluid ist eine kontinuierliche Überwachung, beispielsweise vom Arzt oder bei klinischen Studien, möglich. Durch Abfrage der Überwachungseinrichtung bzw. des Speichers ist damit feststellbar, wann Austräge erfolgten und ggf. welche Mengen vom Zerstäuber abgegeben wurden.

Eine Blockierung einer wiederholten Betätigung des Zerstäubers innerhalb einer vorbestimmten, vorzugsweise vorgebbaren und speicherbaren Mindestzeitdauer kann eine Überdosierung des Fluids, bei dem es sich ja vorzugsweise um ein hochwirksames Arzneimittel handelt, verhindern.

Durch Ausgabe eines Erinnerungssignals vorzugsweise zur erneuten Betätigung des Zerstäubers beispielsweise nach Ablauf einer vorbestimmten, vorzugsweise vorgebbaren und speicherbaren Höchstzeitdauer kann ein Benutzer daran erinnert werden, daß eine erneute Inhalation erfolgen sollte. So kann eine regelmäßige Inhalation, also Aufnahme des Fluids, unterstützt werden. Insbesondere kann das Erinnerungssignal die Zeit bis zur nächsten Inhalation bzw. Betätigung oder ggf. einer überfälligen Inhalation bzw. Betätigung anzeigen. Besonders bevorzugt ist das Erinnerungssignal ein Vorwarn- oder Alarmsignal insbesondere wie in der WO 03/092576 A2 offenbart, die hiermit vollumfänglich als ergänzende Offenbarung eingeführt wird.

Weitere Vorteile, Merkmale, Eigenschaften und Aspekte der vorliegenden Erfindung ergeben sich aus der folgenden Beschreibung bevorzugter Ausführungsformen anhand der Zeichnung. Es zeigt:
- Fig. 1: einen schematischen Schnitt eines bekannten Zerstäubers im ungespannten Zustand;
- Fig. 2: einen schematischen, um 90° gegenüber Fig. 1 gedrehten Schnitt des bekannten Zerstäubers im gespannten Zustand;
- Fig. 3: eine schematische Schnittdarstellung eines unteren Gehäuseteils eines vorschlagsgemäßen Zerstäubers mit integrierter Überwachungseinrichtung;
- Fig. 4: eine blockschaltbildartige Darstellung der Überwachungseinrichtung;
- Fig. 5: eine schematische Darstellung einer Anschlußeinrichtung für die Überwachungseinrichtung; und
- Fig. 6: einen schematischen Schnitt eines anderen vorschlagsgemäßen Zerstäubers mit einem Aufnahmesensor und einem Spraysensor.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

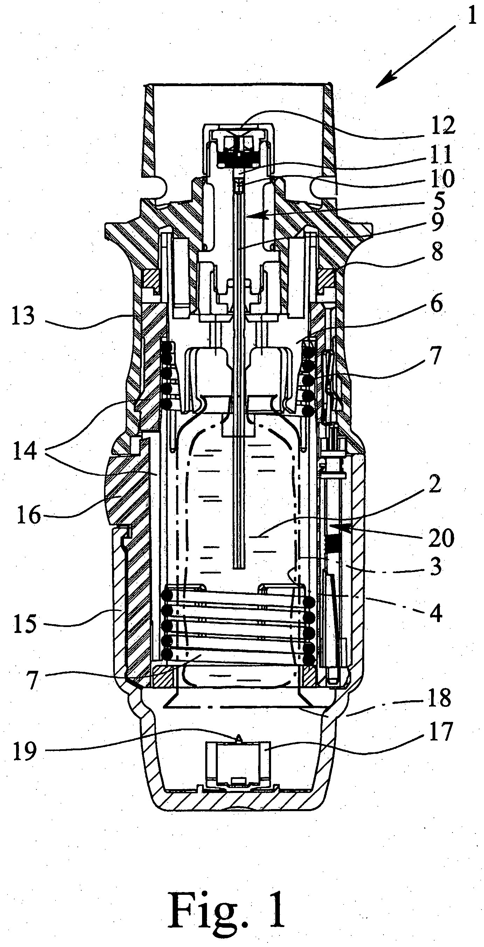

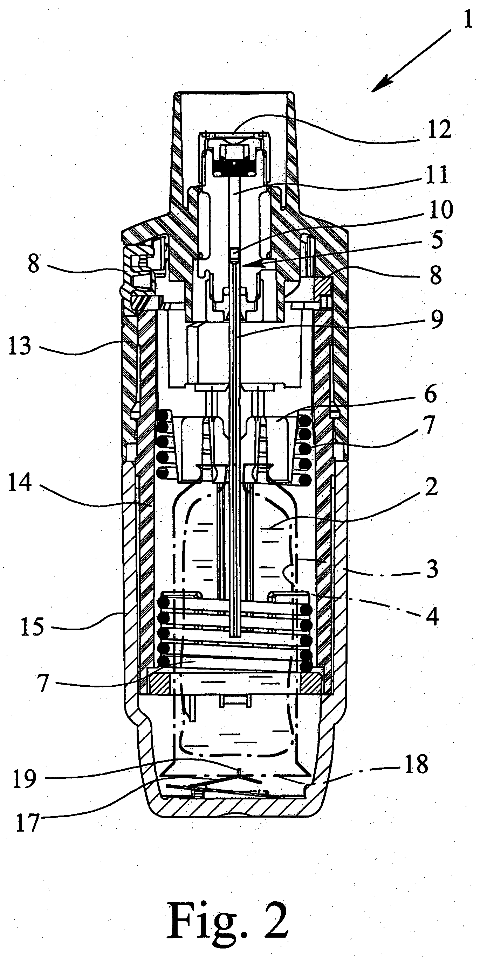

Fig. 1 und 2 zeigen einen bekannten Zerstäuber 1 zur Zerstäubung eines Fluids 2, insbesondere eines hochwirksamen Arzneimittels o.dgl., in einer schematischen Darstellung im ungespannten Zustand (Fig. 1) und gespannten Zustand (Fig. 2). Der Zerstäuber 1 ist insbesondere als tragbarer Inhalator ausgebildet und arbeitet vorzugsweise ohne Treibgas.

Bei Zerstäubung des Fluids 2, vorzugsweise einer Flüssigkeit, insbesondere eines Arzneimittels, wird ein Aerosol gebildet, das von einem nicht dargestellten Benutzer eingeatmet bzw. inhaliert werden kann. Üblicherweise erfolgt das Inhalieren wenigstens einmal täglich, insbesondere mehrmals täglich, vorzugsweise in vorbestimmten Zeitabständen.

Der Zerstäuber 1 weist einen einsetzbaren und vorzugsweise wechselbaren Behälter 3 mit dem Fluid 2 auf, der ein Reservoir für das zu zerstäubende Fluid 2 bildet. Vorzugsweise enthält der Behälter 3 eine ausreichende Menge an Fluid 2 für eine mehrfache Anwendung, insbesondere für eine vorbestimmte Applikationszeit, wie einen Monat, oder für mindestens 50, vorzugsweise mindestens 100, Dosierungen bzw. Zerstäubungen.

Der Behälter 3 ist im wesentlichen zylindrisch bzw. kartuschenartig ausgebildet und von unten, nach Öffnen des Zerstäubers 1, in diesen einsetzbar und ggf. wechselbar. Er ist vorzugsweise starr ausgebildet, insbesondere wobei das Fluid 2 in einem Beutel 4 im Behälter 3 aufgenommen ist.

Der Zerstäuber 1 weist einen Druckerzeuger 5 zur Förderung und Zerstäubung des Fluids 2, insbesondere jeweils in einer vorbestimmten, ggf. einstellbaren Dosiermenge, auf. Der Druckerzeuger 5 weist eine Halterung 6 für den Behälter 3, eine zugeordnete Antriebsfeder 7 mit einem zur Entsperrung manuell betätigbaren Sperrelement 8, ein Förderrohr 9 mit einem Rückschlagventil 10, eine Druckkammer 11 und eine Austragsdüse 12 auf.

Beim axialen Spannen der Antriebsfeder 7 wird die Halterung 6 mit dem Behälter 3 und dem Förderrohr 9 bei den Darstellungen nach unten bewegt und Fluid 2 aus dem Behälter 3 in die Druckkammer 11 des Druckerzeugers 5 über das Rückschlagventil 10 gesaugt. Da die Austragsdüse 12 einen sehr geringen Strömungsquerschnitt hat und insbesondere als Kapillare ausgebildet ist, ergibt sich eine so starke Drosselwirkung, daß auch ohne Rückschlagventil an dieser Stelle ein Einsaugen von Luft sicher ausgeschlossen ist.

Beim anschließenden Entspannen nach Betätigung des Sperrelements 8 wird das Fluid 2 in der Druckkammer 11 von der das Förderrohr 9 wieder nach oben bewegenden Antriebsfeder 7 - also durch Federkraft - unter Druck gesetzt und über die Austragsdüse 12 ausgegeben, wobei es zerstäubt wird, insbesondere in Partikel im µm- oder nm-Bereich, vorzugsweise lungengängige Partikel mit etwa 5 µm. Die Förderung und Zerstäubung des Fluids 2 erfolgen vorzugsweise also rein mechanisch, insbesondere ohne Treibgas und ohne Elektrik.

Der Zerstäuber 1 weist ein Gehäuseoberteil 13 und ein demgegenüber drehbares Innenteil 14 auf, an dem ein Betätigungsteil 15 vorzugsweise mittels eines Halteelementes 16 lösbar befestigt, insbesondere aufgesteckt, ist. Zum Einsetzen und/oder Auswechseln des Behälters 3 ist das Betätigungsteil 15 vom Zerstäuber 1 lösbar.

Durch manuelles Drehen des Betätigungsteils 15 ist das Innenteil 14 relativ zum Gehäuseoberteil 13 drehbar, wodurch die Antriebsfeder 7 über ein nicht dargestelltes, auf die Halterung 6 wirkendes Getriebe in axialer Richtung spannbar ist. Beim Spannen wird der Behälter 3 axial nach unten bewegt, bis der Behälter 3 eine in Fig. 2 angedeutete Endlage im gespannten Zustand annimmt. Während des Zerstäubungsvorgangs wird der Behälter 3 von der Antriebsfeder 7 wieder in seine Ausgangslage zurückbewegt. Das axiale Bewegen des Behälters 3 beim Betätigen des Zerstäubers 1 wird nachfolgend auch als Hub des Behälters 3 bezeichnet.

Das Betätigungsteil 15 bildet vorzugsweise ein klappenartiges Gehäuseunterteil und um- bzw. übergreift einen unteren freien Endbereich des Behälters 3. Beim Spannen der Antriebsfeder 7 bewegt sich der Behälter 3 mit seinem Endbereich (weiter) in das Betätigungsteil 15 bzw. zu dessen stirnseitigem Ende hin, wobei eine axial wirkende, im Betätigungsteil 15 angeordnete Feder 17 am Behälterboden 18 zur Anlage kommt und mit einem Anstechelement 19 den Behälter 3 bei der erstmaligen Anlage zur Belüflung ansticht.

Der Zerstäuber 1 weist eine Überwachungseinrichtung 20 auf, die Betätigungen des Zerstäubers 1 zählt, indem sie ein Drehen des Innenteils 14 zum Gehäuseoberteil 13 erfaßt. Die Überwachungseinrichtung 20 arbeitet rein mechanisch.

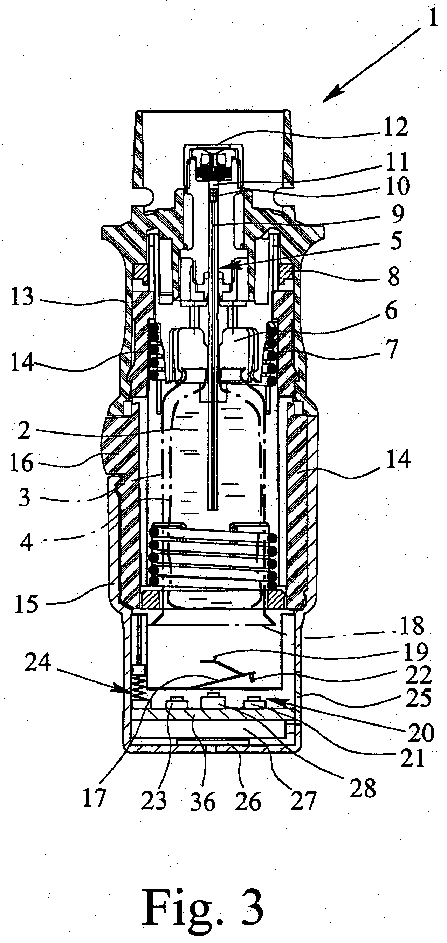

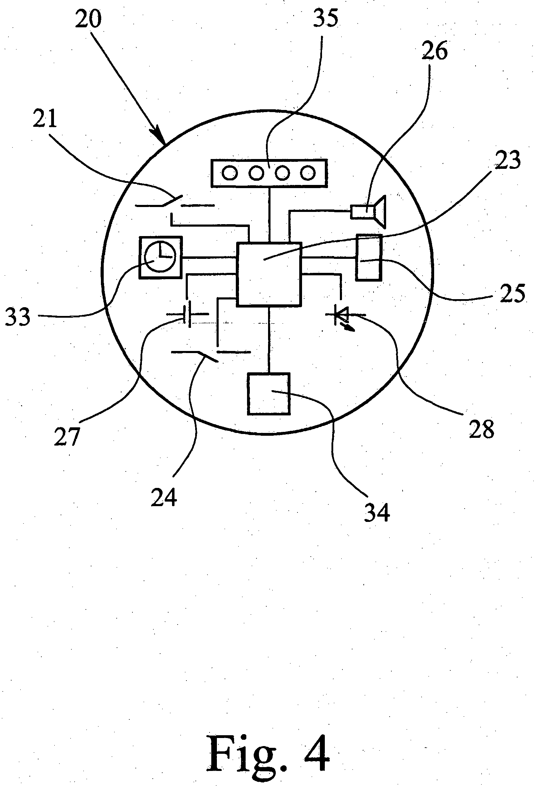

Nachfolgend werden der Aufbau und die Funktionsweise eines vorschlagsgemäßen Zerstäuber 1 mit einer modifizierten Überwachungseinrichtung 20 näher erläutert, wobei auf die Schnittansicht gemäß Fig. 3 und den blockschaltbildartigen Aufbau gemäß Fig. 4 Bezug genommen wird und im übrigen die Ausführungen zu Fig. 1 und 2 gelten.

Die Überwachungseinrichtung 20 ist vorzugsweise in ein lösbares und vorzugsweise auswechselbares Gehäuseteil des Zerstäubers 1, insbesondere in das Betätigungsteil 15 des Zerstäubers 1, eingebaut. Die Überwachungseinrichtung 20 ist vorzugsweise im Bereich des axialen Endes des Zerstäubers 1 bzw. des Betätigungsteils 15 angeordnet, insbesondere eingegossen.

Bei eingesetztem Behälter 3 ist die Überwachungseinrichtung 20 vorzugsweise benachbart zu dem Behälterboden 18 des Behälters 3 und/oder in Verlängerung der Bewegungs- bzw. Hubrichtung des Behälters 3 angeordnet.

Die Überwachungseinrichtung 20 erfaßt als tatsächlichen Austrag von Fluid 2 vorzugsweise Bewegungen bzw. Hübe des Behälters 3 vorzugsweise mechanisch, optisch, elektrisch, induktiv, kapazitiv und/oder anderweit berührungslos. Insbesondere weist die Überwachungseinrichtung 20 gemäß dem Darstellungsbeispiel einen Mikroschalter 21 oder sonstigen Schalter, beispielsweise einen Näherungsschalter, induktiven Schalter, kapazitiven Schalter oder Reed-Kontakt, oder geeigneten Sensor auf.

Der hier konkret vorgesehene Mikroschalter 21 ist hier von einem Vorsprung 22 der Feder 17 betätigbar. Insbesondere drückt der Behälter 3 in seiner unteren Endlage - also bei gespanntem Zerstäuber 1 bzw. Druckerzeuger 5 - die Feder 17 derart nieder, daß der Vorsprung 22 den Mikroschalter 21 betätigt.

Beim Darstellungsbeispiel verlaufen die Bewegungen bzw. Hübe des Behälters 3 axial bzw. linear. Jedoch kann von der Überwachungseinrichtung 20 alternativ oder zusätzlich auch eine nicht lineare oder nicht axiale Bewegung des Behälters 3 bei anderer Ausgestaltung des Zerstäubers 1 und/oder eine Bewegung eines sonstigen Teils des Zerstäubers 1, insbesondere bei dessen Betätigung, erfaßbar sein. Beispielsweise kann die Überwachungseinrichtung 20 alternativ oder zusätzlich gemäß einer nicht dargestellten Ausführungsvariante eine Betätigung des Zerstäubers 1 bzw. einen tatsächlichen Austrag von Fluid 2 durch Messung der Impedanz der Feder 17, die sich in Abhängigkeit von der Spannlage ändert, erfassen.

Die Überwachungseinrichtung 20 erfaßt vorzugsweise, wenn der Behälter 3 die Endlage im gespannten Zustand erreicht und/oder wenn er diese während des Zerstäubungsvorgangs verläßt, als Betätigung des Zerstäubers 1, die gezählt wird. Insbesondere weist die Überwachungseinrichtung 20 eine Steuereinheit 23, vorzugsweise einen Microcontroller o.dgl. auf, um das genannte Zählen und/oder sonstige Funktionen der Überwachungseinrichtung 20 zu realisieren. An die Steuereinheit 23 sind die anderen Komponenten der Überwachungseinrichtung 20 angeschlossen.

Alternativ oder zusätzlich kann die Überwachungseinrichtung 20 auch Bewegungen des Behälters 3 oder eines sonstigen Teils des Zerstäubers 1, wie der Feder 17 oder der Halterung 6, erfassen und insbesondere auswerten. Vorzugsweise werden hierbei die Lage bzw. Position, Geschwindigkeit, diesbezügliche Parameter und insbesondere eine Weg-Zeit-Kurve oder dgl. erfaßt und ausgewertet.

Zusätzlich erfaßt die Überwachungseinrichtung 20 beim Zählen auch den Zeitpunkt der Betätigung, worauf später noch näher eingegangen wird.

Die Hübe des Behälters 3 stellen also einen Zählwert für die Anzahl der Betätigungen des Zerstäubers 1 und damit für die ausgegebene Menge an Fluid 2 dar. Der Zählwert indiziert also den Füllstand des Fluids 2.

Der Zählwert der Betätigungen des Zerstäubers 1, wobei wahlweise die Anzahl der erfolgten Betätigungen und/oder die Anzahl der noch möglichen Betätigungen mit dem aktuellen Behälter 3 anzeigbar und/oder speicherbar ist bzw. sind, ist vorzugsweise manuell oder selbsttätig, insbesondere beim Wechsel des Behälters 3, rücksetzbar. Vorzugsweise erfolgt das Rücksetzen des Zählwerts selbsttätig nach dem Aufsetzen bzw. Aufstecken des Betätigungsteils 15, wobei das Aufsetzen bzw. Aufstecken des Betätigungsteils 15 vorzugsweise mittels eines Kontaktschalters 24 o.dgl. von der Überwachungseinrichtung 20 erfaßbar ist.

Beim Darstellungsbeispiel ist der Kontaktschalter 24 mittels eines federvorgespannten Kontaktstifts auslösbar bzw. betätigbar, wobei der Kontaktstift bei aufgesetztem Betätigungsteil 15 vom Innenteil 14 gegen Federkraft niedergedrückt bzw. eingedrückt wird. Diese Ausführungsvariante hat neben der selbsttätigen Rücksetzung des Zählwerts den weiteren Vorteil, daß bei zusammengebautem Zerstäuber 1 ein (erneutes) Betätigen des Kontaktschalters 24 und ein damit verbundenes Rücksetzen des Zählwerts ausgeschlossen sind. Somit ergibt sich eine einfache, für Bedienungsfehler wenig anfällige Bedienung des Zerstäubers 1.

Der Kontaktschalter 24 kann zusätzlich oder alternativ dazu dienen, die Überwachungseinrichtung 20 einzuschalten bzw. zu aktivieren, insbesondere durch Erfassen des erstmaligen Zusammenbaus des Zerstäubers 1.

Alternativ oder zusätzlich zu dem Kontaktschalter 24 kann ein sonstiger Schalter, wie ein induktiver Schalter, kapazitiver Schalter, Reed-Kontakt, Näherungsschalter o.dgl., oder ein sonstiger geeigneter Sensor eingesetzt werden.

Gemäß einer nicht dargestellten Ausführungsvariante ist der Zerstäuber 1 vorzugsweise derart ausgebildet, daß er nur bei ein- bzw. angebauter Überwachungseinrichtung 20 und/oder nur bei eingeschalteter Überwachungseinrichtung 20 auslösbar bzw. betätigbar ist. Dies kann durch eine entsprechende mechanische und/oder elektrische Verbindung oder Kopplung des Zerstäubers 1 mit der Überwachungseinrichtung 20 oder ggf. mit dem die Überwachungseinrichtung 20 enthaltenden Gehäuseteil bzw. Betätigungsteil 15 realisiert sein.

Insbesondere ist der Zerstäuber 1 bei nicht eingeschalteter Überwachungseinrichtung 20, bei fehlender Überwachungseinrichtung 20, bei fehlendem Betätigungsteil 15 und/oder bei fehlendem Behälter 3 gegen Betätigen gesperrt.

Die Überwachungseinrichtung 20 weist vorzugsweise eine insbesondere optische Anzeigeeinrichtung 25 - beim Darstellungsbeispiel ein Display o.dgl. - auf, insbesondere zur Anzeige des Zustands der Überwachungseinrichtung 20, der vergangenen Zeit nach der letzten Betätigung des Zerstäubers 1, der verbleibenden Zeit bis zur nächsten Betätigung des Zerstäubers 1, der Anzahl der bereits erfolgten Betätigungen des Zerstäubers 1, der Anzahl der noch möglichen Betätigungen des Zerstäubers 1, von noch auszuführenden Betätigungen des Zerstäubers 1 (beispielsweise beim Einsetzen eines neuen Behälters 3), eines erfolgten oder eines erforderlichen Behälterwechsels, des Fluidfüllstands, einer Behälteridentifikation und/oder einer Fluidbezeichnung. Dies gestattet eine optimale Information und ggf. Führung des Benutzers. Somit wird die Handhabung des Zerstäubers 1 erleichtert und eine höhere Bedienungssicherheit ermöglicht.

Die Überwachungseinrichtung 20 weist vorzugsweise eine akustische Anzeigeeinrichtung 26, insbesondere einen Piezosignalgeber o.dgl., auf, insbesondere zur Ausgabe eines Erinnerungssignals zur Erinnerung an eine anstehende Betätigung des Zerstäubers 1 und/oder Anzeige eines andauernden und/oder abgeschlossenen Zerstäubungsvorgangs.

Beispielsweise kann die Überwachungseinrichtung 20 durch Ausgabe eines während des Zerstäubungsvorgangs andauernden Tonsignals dem Benutzer anzeigen, daß eine Zerstäubung erfolgt und der Benutzer das erzeugte Aerosol entsprechend inhalieren soll. Vorzugsweise endet das Tonsignals nicht mit Beendigung des Zerstäubungsvorgangs, sondern erst nach einer darüber hinaus gehenden Zeitdauer, um sicherzustellen, daß der Benutzer das erzeugte Aerosol auch vollständig inhaliert. Aufgrund der verhältnismäßig genau festgelegten Zeitdauer von etwa ein bis zwei Sekunden des Zerstäubungsvorgangs kann die Gesamtzeitdauer des genannten Tonsignals unabhängig vom tatsächlichen Zerstäubungsvorgang durch eine vorzugsweise vorgebbare Zeitdauer, von beispielsweise drei bis vier Sekunden oder von etwa 10 bis 15 Sekunden bei Berücksichtigung eines nach dem Inhalieren erwünschten Luftanhaltens festgelegt werden. Die Überwachungseinrichtung 20 gibt dann das Tonsignal nach Auslösen des Zerstäubungsvorgangs durch Betätigen des Sperrelements 8 - also beginnend mit Detektion des Hubs des Behälters 3 in Zerstäubungsrichtung mittels des Mikroschalters 21 - aus.

Alternativ oder zusätzlich zu dem vorgenannten Tonsignal, das fortlaufend oder wiederholend während des tatsächlichen oder anzuzeigenden Zerstäubungsvorgangs ausgeben wird, kann die Überwachungseinrichtung 20 auch ein Endsignal zur Anzeige des Abschlusses des Zerstäubungsvorgangs, beispielsweise nach Ablauf der vorbestimmten Zeitdauer, ausgeben.

Zusätzlich oder alternativ kann insbesondere mittels der akustischen Anzeigeeinrichtung 26 - ggf. mit einem anderen Signal - einem Benutzer die tatsächliche Dauer des Inhalierens und/oder ein ausreichend starkes Inhalieren und/oder ein nicht ausreichend starkes oder nicht ausreichend langes Inhalieren angezeigt werden.

Vorzugsweise erzeugt die akustische Anzeigeeinrichtung 26 ein Schallsignal, das bedarfsweise durch eine im Betätigungsteil 15 vorgesehene Öffnung austreten kann.

Zusätzlich oder alternativ kann die akustische Anzeigeeinrichtung 26 als Signal ein Vibrieren des Betätigungsteils 15 bzw. des Zerstäubers 1 bewirken, also ein Vibrationssignal oder sonstiges taktiles Signal ausgeben.

Die Überwachungseinrichtung 20 weist einen Energiespeicher, insbesondere eine Batterie 27 oder ggf. einen nicht dargestellten Akkumulator, auf. Der Energiespeicher wird vorzugsweise erst bei Einschalten der Überwachungseinrichtung 20 angeschlossen, um eine lange Lagerfähigkeit bei allenfalls minimalem Energieverlust zu ermöglichen.

Vorzugsweise weist der Energiespeicher bzw. die Batterie 27 eine derartige Kapazität auf, daß die Überwachungseinrichtung 20 nach dem Einschalten mindestens ein Jahr, vorzugsweise mindestens zwei Jahre, insbesondere mindestens fünf Jahre, funktionsfähig bleibt.

Die Überwachungseinrichtung 20 weist ferner eine vorzugsweise nur optisch arbeitende Schnittstelle 28 - beim Darstellungsbeispiel eine Leuchtdiode - auf, die insbesondere ein Einschalten, Programmieren, Einstellen, Rücksetzen und/oder Abfragen der Überwachungseinrichtung 20 ermöglicht.

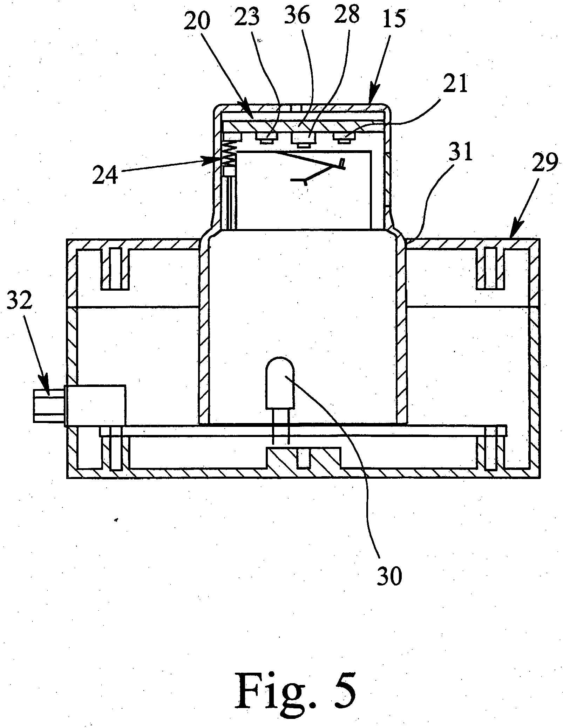

Zur Kommunikation mit der Überwachungseinrichtung 20 über die Schnittstelle 28 ist vorzugsweise eine beispielhaft in Fig. 5 dargestellte Anschlußeinrichtung 29 vorgesehen. Insbesondere ist die Überwachungseinrichtung 20 über die Schnittstelle 28 - ggf. erst nach Trennen der Überwachungseinrichtung 20 zusammen mit dem Gehäuse- bzw. Betätigungsteil 16 vom Zerstäuber 1 - an die Anschlußeinrichtung 29 anschließbar. Vorzugsweise weist die Anschlußeinrichtung 29 zur bevorzugten optischen Kommunikation bzw. Datenübertragung mit der Überwachungseinrichtung 20 eine Leuchtdiode 30 o.dgl. auf.

Zum Anschluß ist die Überwachungseinrichtung 20 bzw. das Betätigungsteil 15 insbesondere in eine entsprechende Aussparung 31 der Anschlußeinrichtung 29 einsetzbar und/oder die Anschlußeinrichtung 29 zumindest teilweise in das Betätigungsteil 15 einführbar.

Die Anschlußeinrichtung 29 ist vorzugsweise an einen nicht dargestellten Computer o. dgl. anschließbar, beispielsweise über einen Anschluß 32. Entsprechend ist die Überwachungseinrichtung 20 sehr einfach initialisierbar, einschaltbar, programmierbar, rücksetzbar, abfragbar o.dgl. Von der Überwachungseinrichtung 20 gespeicherte Daten sind entsprechend einfach abrufbar, anzeigbar und ggf. auswertbar. Dies ist insbesondere für klinische Untersuchungen und/oder eine Patientenüberwachung durch einen Arzt vorteilhaft.

Alternativ oder zusätzlich kann die Anschlußeinrichtung 29 auch unabhängig von einem Computer oder dgl. einsetzbar sein.

Bedarfsweise kann die Anschlußeinrichtung 29 auch ein eigenes Display und/oder eine Tastatur oder sonstige Eingabeeinrichtung aufweisen, um die Überwachungseinrichtung 20 einschalten, programmieren, einstellen, rücksetzen und/oder abfragen zu können. Insbesondere sind dann verschiedene Parameter oder dgl. anzeigbar, wobei in diesem Fall bedarfsweise die optische Anzeigeeinrichtung 25 und/oder eine Eingabeeinrichtung 35 der Überwachungseinrichtung 20 entfallen kann bzw. können.

Statt der Batterie 27 kann bedarfsweise auch ein Akkumulator eingesetzt werden. Ein Aufladen kann dann beispielsweise über einen elektrischen Anschluß oder ggf. induktiv erfolgen, insbesondere gleichzeitig mit dem Anschluß an die Anschlußeinrichtung 29 und/oder über eine nicht dargestellte Solarzelle der Überwachungseinrichtung 20.

Die Überwachungseinrichtung 20 ist vorzugsweise derart ausgebildet, daß sie erst beim erstmaligen Zusammenbau mit dem Zerstäuber 1, beim erstmaligen Einsetzen des Behälters 3, beim erstmaligen Spannen oder Betätigen des Druckerzeugers 5 und/oder beim Initialisieren über die Schnittstelle 28 eingeschaltet wird. Dies ist im Hinblick auf eine lange Lagerfähigkeit vorteilhaft.

Vorzugsweise ist die Überwachungseinrichtung 20 nach dem Einschalten nicht mehr abschaltbar. Dies gewährleistet die gewünschte fortlaufende Überwachung.

Die Überwachungseinrichtung 20 weist vorzugsweise einen Zeitgeber 33 und einen Speicher 34 auf, insbesondere so daß Anzahl und Zeitpunkt der Betätigungen des Zerstäubers 1 bzw. des Austrags von Fluid 2 erfaßbar und speicherbar sind und/oder so daß eine wiederholte Betätigung des Zerstäubers 1 innerhalb einer vorbestimmten Mindestzeitdauer blockierbar ist und/oder so daß nach Ablauf einer vorbestimmten Höchstzeitdauer das bereits genannte Erinnerungssignal zur erneuten Betätigung des Zerstäubers 1 ausgebbar ist.

Wenn die Anzahl und Zeitpunkte der Betätigungen bzw. des Austrags von Fluid 2 fortlaufend im Speicher 34 gespeichert werden, kann durch Abfrage der Überwachungseinrichtung 20 - also Auslesen des Speichers 34 - festgestellt und überwacht werden, wann und in welchem Umfang der Zerstäuber 1 benutzt bzw. Fluid 2 ausgegeben worden ist. Die Überwachungsmöglichkeit ist für den Benutzer zur Selbstkontrolle und/oder für eine Überwachung durch den behandelnden Arzt und/oder für klinische Studien - insbesondere zur Überwachung der Einhaltung von verordneten Dosierungen des Fluids 2 - vorteilhaft.

Wenn eine wiederholte Betätigung des Zerstäubers 1 innerhalb einer vorbestimmten Mindestzeitdauer blockierbar ist, kann eine Überdosierung vermieden werden.

Der Speicher 34 weist vorzugsweise einen EPROM oder EEPROM auf, wobei im letzteren Fall ein elektrisches Rücksetzen möglich ist.

Der Speicher 34 bzw. dessen Inhalt ist vorzugsweise zumindest für den Benutzer bzw. Patienten nicht löschbar oder veränderbar.

Vorzugsweise stellt der Zeitgeber 33 keine absolute Zeitbäsis dar; vielmehr handelt es sich hierbei um einen (einfachen) Zähler, der nur eine relative Zeit erfaßt bzw. zur Verfügung stellt. Dies ermöglicht einen besonders einfachen und kostengünstigen Aufbau.

Der absolute Beginn der relativen Zeiterfassung durch den Zeitgeber 33 wird vorzugsweise beim Initialisieren oder erstmaligen Einschalten der Überwachungseinrichtung 20 festgelegt und ist insbesondere im Speicher 34 oder von einer sonstigen Einrichtung, wie dem nicht dargestellten Computer zur Initialisierung der Überwachungseinrichtung 20, gespeichert.

Alternativ oder zusätzlich kann auch das absolute Ende der relativen Zeiterfassung durch den Zeitgeber 33 einfach beim Abfragen der Überwachungseinrichtung 20 durch Vergleich mit einer absoluten Zeitbasis festgelegt werden. So können die absoluten Zeiten der von der Überwachungseinrichtung 20 erfaßten und im Speicher 34 gespeicherten Betätigungen des Zerstäubers 1 festgelegt bzw. festgestellt werden.

Gemäß einer bevorzugten Ausführungsvariante ist bzw. sind der Zerstäuber 1 und/oder die Überwachungseinrichtung 20 derart ausgebildet, daß der eingesetzte Behälter 3 und/oder dessen Füllgrad und/oder dessen Fluid 2 von der Überwachungseinrichtung 20 vorzugsweise selbsttätig identifizierbar und die Behälteridentifizierung insbesondere abspeicherbar und/oder anzeigbar ist. So ist nachvollziehbar, welcher Behälter 3 und damit welches Fluid 2 verwendet worden sind.

Beispielsweise kann die Überwachungseinrichtung 20 bzw. der Zerstäuber 1 einen nicht dargestellten Barcode-Leser oder eine sonstige Einrichtung zur Abfrage einer Identifizierung bzw. Kodierung, wie eines Barcodes, des Behälters 3 aufweisen. So ist beispielsweise feststellbar, ob ein falscher Behälter 3 oder ein Behälter 3 mit einem falschen Fluid 2, einer falschen Fluidmenge und/oder einer falschen Wirkstoffkonzentration des Fluids 2 eingesetzt wird. Je nach Programmierung bzw. Einstellung der Überwachungseinrichtung 20 kann dann eine Betätigung des Zerstäubers 1 auch sperrbar sein.

Vorzugsweise weist die Überwachungseinrichtung 20 eine abfragbare Identifikation zur Identifizierung der Überwachungseinrichtung 20, des Zerstäubers 1 und/oder eines Benutzers auf.

Die Überwachungseinrichtung 20 weist vorzugsweise alternativ oder zusätzlich zur Schnittstelle 28 eine manuell betätigbare Eingabeeinrichtung 35, insbesondere einen Taster, eine Tastatur o. dgl., auf. Vorzugsweise ist mittels der Eingabeeinrichtung 35 die Überwachungseinrichtung 20 einschaltbar, programmierbar, einstellbar, rücksetzbar und/oder abfragbar. Beispielsweise können mittels der Eingabeeinrichtung 35 die Mindestzeitdauer, die Höchstzeitdauer und/oder die Anzahl der Dosierung, beispielsweise die Anzahl der Hübe pro Anwendung und die Anzahl der Anwendungen pro Tag, eingestellt werden.

Alle oder zumindest die meisten Komponenten der Überwachungseinrichtung 20 sind vorzugsweise auf einer Leiterplatte 36 angeordnet und/oder an diese angeschlossen. Insbesondere bildet die Überwachungseinrichtung 20 eine Baugruppe, die in das Gehäuse- bzw. Betätigungsteil 15 des Zerstäubers 1 eingesetzt, vorzugsweise eingegossen, ist.

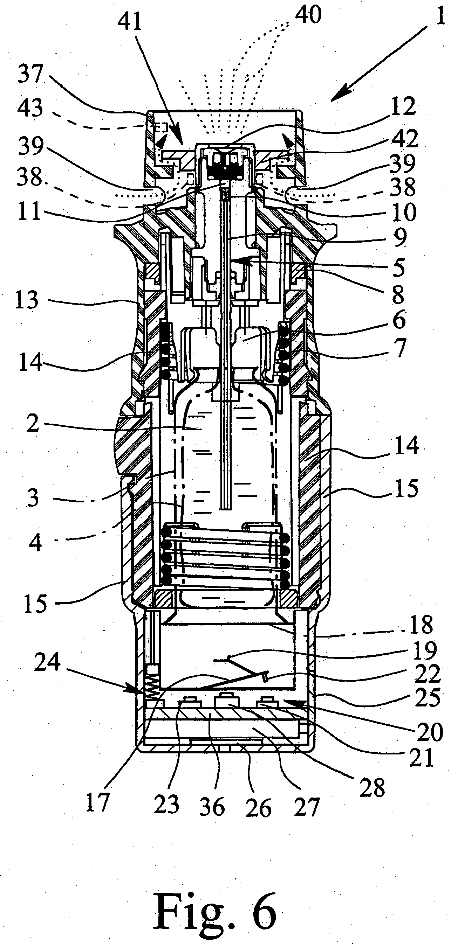

Gemäß einem weiteren, bedarfsweise auch unabhängig realisierbaren Aspekt weist der vorschlagsgemäße Zerstäuber 1 insbesondere im Bereich eines Mundstücks 37 bzw. der Austragsdüse 12 einen Aufnahmesensor 38 zur Erfassung eines Luftstroms und/oder eines Inhalierens von zerstäubtem Fluid 2 auf, wie in Fig. 6 angedeutet.

Vorzugsweise ist der Aufnahmesensor 38 einer Zuluftöffnung 39 zugeordnet, über die insbesondere seitlich oder im Bereich der Austragsdüse 12 Zuluft von einem Benutzer beim Inhalieren ansaugbar ist, wie durch Pfeile in Fig. 6 angedeutet.

Der Aufnahmesensor 38 ist gemäß einer ersten Ausführungsvariante vorzugsweise unmittelbar zur Erfassung eines entsprechenden Zuluftstroms ausgebildet, so daß ein Inhalieren des in Fig. 6 schematisch angedeuteten Aerosols 40 detektiert werden kann.

Vorzugsweise kann der Aufnahmesensor 38 die Richtung eines Luftstroms durch die Zuluftöffnung 39, die Strömungsgeschwindigkeit und/oder den Volumenstrom erfassen. Hierzu kann der Aufnahmesensor 38 beispielsweise als sogenannter Strömungssensor zur unmittelbaren Erfassung einer Luftströmung ausgebildet oder beispielsweise einer nicht dargestellten, vorzugsweise frei schwenkbaren Klappe, einem vom Luftstrom drehbaren Flügelrad oder dgl. zugeordnet sein.

Gemäß einer besonders bevorzugten, bedarfsweise auch unabhängig realisierbaren zweiten Ausführungsvariante ist der Zuluftöffnung 39 bzw. den Zuluftöffnungen 39 ein in Fig. 6 schematisch angedeutetes, insbesondere als Einweg- oder Rückschlagventil ausgebildetes Ventil 41 vorzugsweise mit einem beweglichen Ventilelement 42 zugeordnet, um ein (ungewolltes) Ausblasen des Aerosols 40 durch die Zuluftöffnung(en) 39 verhindern zu können. In diesem Fall erfaßt der Aufnahmesensor 38 vorzugsweise unmittelbar nur das Öffnen und/oder Schließen des Ventils 41, also den Zuluftstrom nur indirekt. Dies ermöglicht einen besonders einfachen und kostengünstigen Aufbau, insbesondere wenn der Aufnahmesensor 38 beispielsweise einen nicht dargestellten Mikroschalter aufweist, der vom Ventilelement 42 betätigbar ist.

Der Aufnahmesensor 38 ist vorzugsweise an die Überwachungseinrichtung 20 angeschlossen, die die Signale des Aufnahmesensors 38 entsprechend auswertet und insbesondere speichert. Dies kann bedarfsweise drahtlos oder sogar mechanisch erfolgen. Vorzugsweise ist der Aufnahmesensor 38 elektrisch über nicht dargestellte Leitungen und geeignete Kontakte am Übergang zum Betätigungsteil 15 an die Überwachungseinrichtung 20 angeschlossen.

Mittels des Aufnahmesensors 38 ist der Zuluftstrom und damit ein tatsächliches Inhalieren des mit dem Zerstäuber 1 erzeugten Aerosols 40 erfaßbar. Dementsprechend kann die Überwachungseinrichtung 20 alternativ oder zusätzlich zu der Erfassung der Bewegung des Behälters 3 ein mittels des Aufnahmesensors 38 erfaßtes Inhalieren des Aerosols 40 als tatsächlichen Austrag oder Aufnahme des Fluids 2 bzw. (erfolgreiche) Betätigung des Zerstäubers 1 zählen und diesen Zählwert im bereits beschriebenen Sinne verarbeiten, anzeigen, speichern o. dgl.

Die mittels des Aufnahmesensors 38 mögliche Erfassung einer tatsächlichen Inhalation des Fluids 2 kann von der Überwachungseinrichtung 20 dahingehend ausgewertet werden, ob ein ausreichend langes Inhalieren erfolgt, wobei die Inhalationszeit bedarfsweise gespeichert und/oder angezeigt werden kann.

Gemäß einer weiteren Ausführungsvariante weist der Zerstäuber 1 zusätzlich oder alternativ zum Aufnahmesensor 38 einen Spraysensor 43 auf, der eine Erfassung ermöglicht, ob tatsächlich eine Zerstäubung erfolgt. Insbesondere erfaßt der Spraysensor, ob sich tatsächlich Tröpfchen des zerstäubten Fluids 2 bzw. Aerosol 40 im Bereich des Mundstücks 37 bilden bzw. bildet. Beispielsweise nutzt der Sensor den Effekt, daß das zerstäubte Fluid 2 bzw. Aerosol 40 Licht streut, und arbeitet als sogenannter Streulicht-Sensor.

Mittels des Spraysensors 43 ist also erfaßbar, ob tatsächlich eine Zerstäubung erfolgt. Insbesondere überprüft die Überwachungseinrichtung 20, ob bei Betätigung des Zerstäubers 1 - vorzugsweise innerhalb eines vorbestimmten Zeitfensters - tatsächlich auch eine Zerstäubung mittels des genannten Sensors erfaßt wird, wobei nur bei Erfassung einer tatsächlichen Zerstäubung das Betätigen des Zerstäubers 1 tatsächlich als Austrag von Fluid 2 und damit als Zerstäubungsvorgang erfaßt bzw. gewählt wird.

Alternativ oder zusätzlich zur Erfassung einer Bewegung des Behälters 3 oder eines sonstigen Teils kann die Überwachungseinrichtung 20 mittels des Aufnahmesensors 38 einen Zuluftstrom und/oder mittels des Spraysensors 43 eine Erzeugung von Aerosol 40 detektieren und dies - ggf. auch nur bei kumulativem Auftreten - als tatsächlichen Austrag von Fluid 2 auswerten und als Betätigung des Zerstäubers 1 zählen und ggf. - insbesondere zusammen mit dem jeweiligen Zeitpunkt - speichern.

### Bezugszeichenliste

- 1: Zerstäuber
- 2: Fluid
- 3: Behälter
- 4: Beutel
- 5: Druckerzeuger
- 6: Halterung
- 7: Antriebsfeder
- 8: Sperrelement
- 9: Förderrohr
- 10: Rückschlagventil
- 11: Druckkammer
- 12: Austragsdüse
- 13: Gehäuseoberteil
- 14: Innenteil
- 15: Betätigungsteil
- 16: Halteelement
- 17: Feder
- 18: Behälterboden
- 19: Anstechelement
- 20: Überwachungseinrichtung
- 21: Mikroschalter
- 22: Vorsprung
- 23: Steuereinheit
- 24: Kontaktschalter
- 25: Anzeigeeinrichtung optisch
- 26: Anzeigeeinrichtung akustisch
- 27: Batterie
- 28: Schnittstelle
- 29: Anschlußeinrichtung
- 30: Leuchtdiode
- 31: Aussparung
- 32: Anschluß
- 33: Zeitgeber
- 34: Speicher
- 35: Eingabeeinrichtung
- 36: Leiterplatte
- 37: Mundstück
- 38: Aufnahmesensor
- 39: Zuluftöffnung
- 40: Aerosol
- 41: Ventil
- 42: Ventilelement
- 43: Spraysensor

## Patentansprüche

1. Zerstäuber (1) für ein Fluid (2), mit einem vorzugsweise einsetzbaren und ggf. wechselbaren Behälter (3) mit dem Fluid (2), mit einem Druckerzeuger (5) zur Förderung und/oder Zerstäubung des Fluids (2) und mit einer Überwachungseinrichtung (20) zur Zählung von Betätigungen des Zerstäubers (1),
**dadurch gekennzeichnet,**
**dass** die Überwachungseinrichtung (20) derart ausgebildet ist, dass ein tatsächlicher Austrag von Fluid (2) erfassbar und als Betätigung des Zerstäubers (1) zählbar ist, wobei der Zerstäuber (1) einen Aufnahmesensor (38) zur Erfassung eines Luftstroms und/oder eines Inhalierens von zerstäubtem Fluid (2) aufweist und wobei der Zerstäuber (1) einen Spraysensor zur Erfassung, ob tatsächlich eine Zerstäubung erfolgt, aufweist.

2. Zerstäuber nach Anspruch 1, **dadurch gekennzeichnet, dass** der tatsächliche Austrag elektronisch als Betätigung des Zerstäubers (1) zählbar ist, und/oder dass die Überwachungseinrichtung (20) einen Zeitgeber (33) und einen Speicher (34) aufweist, insbesondere so dass Anzahl und Zeitpunkt der Betätigung des Zerstäubers (1) erfassbar und speicherbar sind.

3. Zerstäuber nach einem der voranstehenden Ansprüche, **dadurch gekennzeichnet, dass** eine wiederholte Betätigung des Zerstäubers (1) innerhalb einer vorbestimmten Mindestzeitdauer blockierbar ist; und/oder dass vorzugsweise nach Ablauf einer vorbestimmten Höchstzeitdauer ein Erinnerungssignal vorzugsweise zur erneuten Betätigung des Zerstäubers (1) ausgebbar oder anzeigbar ist.

4. Zerstäuber nach einem der voranstehenden Ansprüche, **dadurch gekennzeichnet, dass** der Zerstäuber (1) ein Betätigungsteil (15) aufweist, und/oder, dass die Überwachungseinrichtung (20) am Betätigungsteil (15) angeordnet, insbesondere darin eingebaut, ist; und/oder
dass die Überwachungseinrichtung (20) in Verlängerung einer Bewegungsrichtung des Behälters (3) angeordnet ist.

5. Zerstäuber nach einem der voranstehenden Ansprüche, **dadurch gekennzeichnet, dass** die Überwachungseinrichtung (20) eine vorzugsweise optische Anzeigeeinrichtung (25) aufweist, insbesondere zur Anzeige der vergangenen Zeit nach der letzten Betätigung des Zerstäubers (1), der verbleibenden Zeit bis zur nächsten Betätigung des Zerstäubers (1), der Anzahl der bereits erfolgten Betätigungen des Zerstäubers (1), der Anzahl der noch möglichen Betätigungen des Zerstäubers (1), von noch auszuführenden Betätigungen des Zerstäubers (1), eines erfolgten oder erforderlichen Behälterwechsels, des Fluidfüllstands, einer Behälteridentifikation und/oder einer Fluidbezeichnung.

6. Zerstäuber nach einem der voranstehenden Ansprüche, **dadurch gekennzeichnet, dass** die Überwachungseinrichtung (20) eine akustische Anzeigeeinrichtung (26), vorzugsweise einen Piezosignalgeber, aufweist, insbesondere zur Ausgabe eines Erinnerungssignals zur Betätigung des Zerstäubers (1) und/oder zur Anzeige eines andauernden und/oder abgeschlossenen Zerstäubungsvorgangs, und/oder dass die Überwachungseinrichtung (20) einen Energiespeicher, insbesondere eine Batterie (27), aufweist, der vorzugsweise erst bei Einschalten der Überwachungseinrichtung (20) angeschlossen wird, insbesondere wobei die Batterie (27) eine derartige Kapazität aufweist, dass die Überwachungseinrichtung (20) nach dem Einschalten mindestens ein Jahr, vorzugsweise mindestens zwei Jahre, insbesondere mindestens fünf Jahre, funktionsfähig bleibt.

7. Zerstäuber nach einem der voranstehenden Ansprüche, **dadurch gekennzeichnet, dass** die Überwachungseinrichtung (20) eine vorzugsweise nur optische Schnittstelle (28), vorzugsweise eine Leuchtdiode, insbesondere zum Einschalten, Initialisieren, Programmieren, Einstellen, Rücksetzen und/oder Abfragen der Überwachungseinrichtung (20), aufweist, und/oder dass die Überwachungseinrichtung (20) derart ausgebildet ist, dass sie erst beim Zusammenbau mit dem Zerstäuber (1), beim erstmaligen Einsetzen des Behälters (3), beim erstmaligen Spannen oder Betätigen des Druckerzeugers (5) und/oder Initialisieren über eine Schnittstelle (28) eingeschaltet wird, und/oder dass die Überwachungseinrichtung (20) derart ausgebildet ist, dass sie nach dem Einschalten nicht mehr abschaltbar ist.

8. Zerstäuber nach einem der Ansprüche 2 bis 7, **dadurch gekennzeichnet, dass** mit dem Zeitgeber (33) nur eine relative Zeit erfaßbar ist, insbesondere wobei der absolute Beginn der relativen Zeiterfassung durch den Zeitgeber (33) beim Initialisieren oder erstmaligen Einschalten der Überwachungseinrichtung (20), insbesondere im Speicher (34), speicherbar oder festlegbar ist,
vorzugsweise wobei das absolute Ende der relativen Zeiterfassung durch den Zeitgeber (33) beim Abfragen der Überwachungseinrichtung (20) durch Vergleich mit einer absoluten Zeitbasis feststellbar ist, und/oder, dass der Speicher (34), zumindest für einen Benutzer des Zerstäubers (1), nicht löschbar oder veränderbar ist.

9. Zerstäuber nach einem der voranstehenden Ansprüche, **dadurch gekennzeichnet, dass** die Überwachungseinrichtung (20) eine abfragbare Identifikation zur Identifizierung der Überwachungseinrichtung (20), des Zerstäubers (1) und/oder eines Benutzers aufweist, und/oder dass der Zerstäuber (1) und/oder die Überwachungseinrichtung (20) derart ausgebildet ist bzw. sind, dass der eingesetzte Behälter (3) von der Überwachungseinrichtung (20) vorzugsweise selbsttätig identifizierbar ist und die Behälteridentifizierung insbesondere abspeicherbar ist, und/oder dass die Überwachungseinrichtung (20) eine Eingabeeinrichtung (35), insbesondere zum Einschalten, Initialisieren, Programmieren, Einstellen, Rücksetzen und/oder Abfragen der Überwachungseinrichtung (20), aufweist.

10. Zerstäuber nach einem der voranstehenden Ansprüche, **dadurch gekennzeichnet, dass** der Aufnahmesensor (38) im Bereich eines Mundstücks (37) angeordnet ist, wobei Signale des Aufnahmesensors (38) von der Überwachungseinrichtung (20) erfassbar sind.

11. Zerstäuber nach einem der voranstehenden Ansprüche, **dadurch gekennzeichnet, dass** der Zuluftstrom von einem Benutzer des Zerstäubers (1) beim Inhalieren erzeugbar ist.

12. Zerstäuber nach einem der voranstehenden Ansprüche, **dadurch gekennzeichnet, dass** die Überwachungseinrichtung (20) die Signale des Aufnahmesensors (38) hinsichtlich eines ausreichend starken und/oder ausreichend langen Inhalierens, insbesondere während des jeweiligen Zerstäubungsvorgangs und/oder nach jedem Zerstäubungsvorgang, auswertet, speichert, anzeigt und/oder als erfolgreiche Inhalation von Fluid (2) bzw. tatsächlichen Austrag von Fluid (2) zählt.

13. Zerstäuber nach einem der voranstehenden Ansprüche, **dadurch gekennzeichnet, dass** der Aufnahmesensor (38) als Strömungssensor, insbesondere zur Erfassung einer Strömungsrichtung, der Strömungsgeschwindigkeit, des Volumenstroms und/oder des Massenstroms, ausgebildet ist,

14. Zerstäuber nach einem der voranstehenden Ansprüche, **dadurch gekennzeichnet, dass** der Aufnahmesensor (38) einer Zuluftöffnung (39) des Zerstäubers (1) zugeordnet ist, insbesondere wobei der Zuluftöffnung (39) ein Ventil (41) zugeordnet ist und der Aufnahmesensors (38) eine Bewegung eines Ventilelements (42) des Ventils (41), vorzugsweise mittels eines Mikroschalters, erfasst.

15. Zerstäuber nach einem der voranstehenden Ansprüche, **dadurch gekennzeichnet, dass** der Druckerzeuger (5) nur mechanisch arbeitet und manuell betätigbar, insbesondere gegen Federkraft spannbar, ist, und/oder dass die Druckerzeugung bzw. Zerstäubung rein mechanisch, insbesondere treibgasfrei, vorzugsweise durch Federkraft, erfolgt, und/oder dass der Zerstäuber (1) als Inhalator, insbesondere zur medizinischen Aerosol-Therapie, ausgebildet ist,

## Claims

1. Nebulizer (1) for a fluid (2), comprising a preferably insertable and optional changeable container (3) with the fluid (2), a pressure generator (5) for conveying and/or nebulizing the fluid (2) and a monitoring device (20) for counting the actuations of the nebulizer (1),
**characterized in**
**that** the monitoring device (20) is adapted so that an actual dispensing of fluid (2) can be detected and counted as an actuation of the nebulizer (1), wherein the nebulizer (1) comprises a receiving sensor (38) for detecting an air current and/or an inhaling of nebulized fluid (2), and wherein the nebulizer (1) comprises a spray sensor for detecting whether nebulization has actually occurred.

2. Nebulizer according to claim 1, **characterized in that** the actual dispensing is electronically countable as actuation of the nebulizer (1), and/or that the monitoring device (20) comprises a timer (33) and a memory (34), in particular so that the number and time of the actuation of the nebulizer (1) can be detected and stored.

3. Nebulizer according to any of the preceding claims, **characterized in that** a repeated actuation of the nebulizer (1) within a predetermined minimum period of time can be blocked, and/or that a reminder signal particularly for repeated actuation of the nebulizer (1) can be emitted or displayed, preferably after a predetermined maximum length of time.

4. Nebulizer according to any of the preceding claims, **characterized in that** the nebulizer (1) comprises an actuating member (15), and/or that the monitoring device (20) is mounted on the actuating member (15), in particular incorporated therein, and/or that the monitoring device (20) is arranged in extension of the direction of movement of the container (3).

5. Nebulizer according to any of the preceding claims, **characterized in that** the monitoring device (20) comprises a preferably optical indicator device (25), in particular for indicating the time that has elapsed since the last actuation of the nebulizer (1), the time remaining until the next actuation of the nebulizer (1), the number of actuations of the nebulizer (1) that have already occurred, the number of actuation of the nebulizer (1) that are still possible, the number of actuations of the nebulizer (1) that still have to be carried out, whether a container change has taken place or is necessary, the fill level of fluid, a container identification and/or a designation of fluid.

6. Nebulizer according to any of the preceding claims, **characterized in that** the monitoring device (20) comprises an acoustic indicator device (26), preferably a piezo electric signal transmitter, in particular for emitting a reminder signal for reminding of actuation of the nebulizer (1) and/or for indicating an on-going and/or completed nebulizing process, and/or that the monitoring device (20) comprises an energy storage, in particular a battery (27), that is preferably only connected once the monitoring device (20) has been switched on, in particular wherein the battery (27) has a capacity such that the monitoring device (20) remains operational for at least one year, preferably at least two years, in particular at least five years, after being switched on.

7. Nebulizer according to any of the preceding claims, **characterized in that** the monitoring device (20) comprises a preferably purely optical interface (28), preferably a light emitting diode, in particular for switching on, initializing, programming, setting, resetting, and for querying the monitoring device (20), and/or that the monitoring device (20) is constructed so that it is only switched on when first fitted together with the nebulizer (1), when the container (3) is first inserted, when the pressure generator (5) is first tensioned or actuated and/or when it is initiated through interface (28), and/or that the monitoring device (20) is constructed such that the monitoring device (20) cannot be switched off once it has been switched on.

8. Nebulizer according to any of the claims 2 to 7, **characterized in that** only a relative time can be measured with the timer (33), in particular wherein the absolute start of the relative time measurement by the timer (33) is stored or set when initiating or first switching on the monitoring device (20), preferably in the memory (34), preferably wherein the absolute end of the relative time measurement by timer (33) can be determined by comparison with an absolute time base when querying the monitoring device (20), and/or that the memory (34) cannot be a erased or changed at least for a user of the nebulizer (1).

9. Nebulizer according to any of the preceding claims, **characterized in that** the monitoring device (20) comprises an identifier capable of being queried for identifying the monitoring device (20), the nebulizer (1) and/or a user, and/or that the nebulizer (1) and/or the monitoring device (20) is or are constructed so that the container (3) inserted can be preferably automatically identified by the monitoring device (20) and that the identification of the container can in particular be stored, and/or that the monitoring device (20) comprises an input device (35), in particular for switching on, initiating, programming, setting, resetting and/or querying the monitoring device (20).

10. Nebulizer according to any of the preceding claims, **characterized in that** the receiving sensor (38) is arranged in the region of a mouth piece (37), wherein signals of the receiving sensor (38) can be detected by the monitoring device (20).

11. Nebulizer according to any of the preceding claims, **characterized in that** the air supply current can be produced by a user of the nebulizer (1) during inhaling.

12. Nebulizer according to any of the preceding claims, **characterized in that** the monitoring device (20) evaluates, stores, displaces the signals from the receiving sensor (38) in terms of the sufficient strength or sufficient duration of inhalation, in particular during each nebulizing process and/or after each nebulizing process, and/or counts these signals as successful inhalation of fluid (2) and/or as the actual dispensing of fluid (2).

13. Nebulizer according to any of the preceding claims, **characterized in that** the receiving sensor (38) is constructed as a flow sensor, in particular for detecting a direction of flow, the flow velocity, flow volume or mass flow.

14. Nebulizer according to any of the preceding claims, **characterized in that** the receiving sensor (38) is assigned to an air supply opening (39) of the nebulizer (1), in particular wherein the air supply opening (39) is assigned to a valve (41) and the receiving sensor (38) detects a movement of a valve element (42) of valve (41), preferably via a micro switch.

15. Nebulizer according to any of the preceding claims, **characterized in that** the pressure generator (5) operates only mechanically and is manually actuatable, in particular tensionable against a spring force, and/or that the pressure generation and/or nebulization is carried out only mechanically, in particular without propellant gas, preferably via spring force, and/or that the nebulizer (1) is designed as an inhaler, in particular for medical aerosol therapy.

## Revendications

1. Pulvérisateur (1) pour un fluide (2), comprenant un contenant (3), pouvant être de préférence inséré et éventuellement pouvant être remplacé, renfermant le fluide (2), comprenant un générateur de pression (5) servant à refouler et/ou à pulvériser le fluide (2) et comprenant un dispositif de surveillance (20) servant à compter des actionnements du pulvérisateur (1),
**caractérisé en ce**
**que** le dispositif de surveillance (20) est réalisé de telle manière qu'un déversement réel de fluide (2) peut être détecté et peut être compté en tant qu'actionnement du pulvérisateur (1), le pulvérisateur (1) présentant un capteur d'enregistrement (38) servant à détecter un flux d'air et/ou une inhalation d'un fluide (2) pulvérisé et le pulvérisateur (1) présentant un capteur d'atomisation servant à détecter si une pulvérisation a réellement eu lieu.

2. Pulvérisateur selon la revendication 1, **caractérisé en ce que** le déversement réel peut être compté de manière électronique en tant qu'actionnement du pulvérisateur (1), et/ou **en ce que** le dispositif de surveillance (20) présente une horloge (33) et une mémoire (34), en particulier de telle sorte que le nombre et le moment de l'actionnement du pulvérisateur (1) peuvent être détectés et mémorisés.

3. Pulvérisateur selon l'une quelconque des revendications précédentes, **caractérisé en ce qu'**un actionnement répété du pulvérisateur (1) peut être bloqué dans un laps de temps présentant une durée minimale prédéfinie, et/ou **en ce que** de préférence un signal de rappel de préférence servant à renouveler l'actionnement du pulvérisateur (1) peut être émis ou affiché de préférence au terme d'une période présentant une durée maximale prédéfinie.

4. Pulvérisateur selon l'une quelconque des revendications précédentes, **caractérisé en ce que** le pulvérisateur (1) présente une partie d'actionnement (15), et/ou **en ce que** le dispositif de surveillance (20) est disposé au niveau de la partie d'actionnement (15), en particulier est monté dans cette dernière, et/ou **en ce que** le dispositif de surveillance (20) est disposé dans le prolongement d'une direction de déplacement du contenant (3).

5. Pulvérisateur selon l'une quelconque des revendications précédentes, **caractérisé en ce que** le dispositif de surveillance (20) présente un dispositif d'affichage (25) de préférence optique, en particulier servant à afficher le temps écoulé après le dernier actionnement du pulvérisateur (1), le temps restant jusqu'au prochain actionnement du pulvérisateur (1), le nombre des actionnements ayant déjà eu lieu du pulvérisateur (1), le nombre des actionnements encore possibles du pulvérisateur (1), des actionnements à réaliser encore du pulvérisateur (1), un remplacement de contenant ayant eu lieu ou requis, le niveau de remplissage de fluide, une identification de contenant et/ou une désignation de fluide.

6. Pulvérisateur selon l'une quelconque des revendications précédentes, **caractérisé en ce que** le dispositif de surveillance (20) présente un dispositif d'affichage (26) acoustique, de préférence un émetteur de signal piézoélectrique, en particulier servant à émettre un signal de rappel servant à actionner le pulvérisateur (1) et/ou servant à afficher une opération de pulvérisation continue et/ou finalisée, et/ou **en ce que** le dispositif de surveillance (20) présente un accumulateur d'énergie, en particulier une batterie (27), qui est raccordé de préférence seulement lors de la mise en service du dispositif de surveillance (20), la batterie (27) présentant en particulier une capacité telle que le dispositif de surveillance (20) reste opérationnel après la mise en service au moins pendant un an, de préférence au moins pendant deux ans, en particulier pendant au moins cinq ans.

7. Pulvérisateur selon l'une quelconque des revendications précédentes, **caractérisé en ce que** le dispositif de surveillance (20) présente une interface (28), de préférence seulement optique, de préférence une diode électroluminescente, en particulier servant à mettre en service, à initialiser, à programmer, à régler, à réinitialiser et/ou à interroger le dispositif de surveillance (20), et/ou **en ce que** le dispositif de surveillance (20) est réalisé de telle manière qu'il est mis en service seulement lors de l'assemblage au pulvérisateur (1), lors de la première insertion du contenant (3), lors de la première mise sous contrainte ou du premier actionnement du générateur de pression (5) et/ou lors de l'initialisation par l'intermédiaire d'une interface (28), et/ou **en ce que** le dispositif de surveillance (20) est réalisé de telle manière qu'il ne peut plus être mis hors service après la mise en service.

8. Pulvérisateur selon l'une quelconque des revendications 2 à 7, **caractérisé en ce qu'**une heure relative seulement peut être détectée à l'aide de l'horloge (33), le début absolu de la détection relative de l'heure par l'horloge (33) pouvant être en particulier mémorisée, en particulier dans la mémoire (34), ou pouvant être déterminée lors de l'initialisation ou de la première mise en service du dispositif de surveillance (20), la fin absolue de la détection relative de l'heure pouvant être déterminée de préférence par l'horloge (33) lors de l'interrogation du dispositif de surveillance (20) par comparaison avec une base d'heure absolue, et/ou **en ce que** la mémoire (34), au moins pour un utilisateur du pulvérisateur (1), ne peut être effacée ou modifiée.

9. Pulvérisateur selon l'une quelconque des revendications précédentes, **caractérisé en ce que** le dispositif de surveillance (20) présente une identification pouvant être interrogée servant à identifier le dispositif de surveillance (20), le pulvérisateur (1) et/ou un utilisateur, et/ou **en ce que** le pulvérisateur (1) et/ou le dispositif de surveillance (20) sont réalisés de telle manière que le contenant (3) inséré peut être identifié de préférence en toute autonomie par le dispositif de surveillance (20) et que l'identification de contenant peut être en particulier enregistrée, et/ou **en ce que** le dispositif de surveillance (20) présente un dispositif d'entrée (35), servant en particulier à mettre en service, à initialiser, à programmer, à régler, à réinitialiser et/ou à interroger le dispositif de surveillance (20).

10. Pulvérisateur selon l'une quelconque des revendications précédentes, **caractérisé en ce que** le capteur d'enregistrement (38) est disposé dans la zone d'une embouchure (37), des signaux du capteur d'enregistrement (38) pouvant être détectés par le dispositif de surveillance (20).

11. Pulvérisateur selon l'une quelconque des revendications précédentes, **caractérisé en ce que** le flux d'air entrant peut être généré par un utilisateur du pulvérisateur (1) lors de l'inhalation.

12. Pulvérisateur selon l'une quelconque des revendications précédentes, **caractérisé en ce que** le dispositif de surveillance (20) évalue, mémorise, affiche les signaux du capteur d'enregistrement (38) eu égard à une inhalation suffisamment forte et/ou suffisamment longue, en particulier au cours de l'opération de pulvérisation respective et/ou après chaque opération de pulvérisation et/ou les compte en tant qu'inhalation réalisée avec succès de fluide (2) ou en tant que déversement réel de fluide (2).

13. Pulvérisateur selon l'une quelconque des revendications précédentes, **caractérisé en ce que** le capteur d'enregistrement (38) est réalisé sous la forme d'un capteur d'écoulement, en particulier servant à détecter une direction d'écoulement, la vitesse d'écoulement, le flux volumique et/ou le flux massique.

14. Pulvérisateur selon l'une quelconque des revendications précédentes, **caractérisé en ce que** le capteur d'enregistrement (38) est associé à un orifice d'air entrant (39) du pulvérisateur (1), une soupape (41) étant associée à l'orifice d'air entrant (39) et le capteur d'enregistrement (38) détectant un déplacement de l'élément de soupape (42) de la soupape (41), de préférence au moyen d'un micro-interrupteur.

15. Pulvérisateur selon l'une quelconque des revendications précédentes, **caractérisé en ce que** le générateur de pression (5) fonctionnement seulement de manière mécanique et peut être actionné de manière manuelle, peut être contraint en particulier à l'encontre d'une force de ressort, et/ou **en ce que** la génération de pression ou la pulvérisation est effectuée purement de manière mécanique, en particulier sans gaz propulseur, de préférence par la force de ressort, et/ou **en ce que** le pulvérisateur (1) est réalisé sous la forme d'un inhalateur, en particulier servant à la thérapie médicale par aérosols.
